# FASCICULE DE BREVET EUROPEEN

(11) **EP 3 811 327 B1**
(45) Date de publication et mention de la délivrance du brevet: **03.08.2022**
(21) Numéro de dépôt: 19790599.5
(22) Date de dépôt: 18.06.2019
(51) Int. Cl.: G06T 5/00

(54) **SYSTEME ET PROCEDE POUR LE TRAITEMENT D'AU MOINS UNE REGION POLLUANTE D'UNE IMAGE NUMERIQUE D'UN OBJET EXPOSE A DES RAYONS X**
SYSTEM UND VERFAHREN ZUR VERARBEITUNG MINDESTENS EINES VERUNREINIGTEN BEREICHS EINES DIGITALEN BILDES EINES MIT RÖNTGENSTRAHLEN BESTRAHLTEN OBJEKTS
SYSTEM AND METHOD FOR PROCESSING AT LEAST ONE POLLUTING REGION OF A DIGITAL IMAGE OF AN OBJECT EXPOSED TO X-RAYS

(30) Priorité: 19.06.2018 FR 1855390
(43) Date de publication de la demande: 28.04.2021
(73) Titulaire: HERA-MI, 44800 St Herblain (FR)
(72) Inventeur: TARDY, Mickael, 44000 NANTES (FR); SCHEFFER, Bruno, 44300 NANTES (FR)
(74) Mandataire: Ipsilon
(86) Numéro de dépôt international: PCT/FR2019/051487
(87) Numéro de publication internationale: WO 2019/243731

(56) Documents cités:
- US-A1- 2016 133 033
- HE WENDA ET AL: "Breast image pre-processing for mammographic tissue segmentation", COMPUTERS IN BIOLOGY AND MEDICINE, NEW YORK, NY, US, vol. 67, 14 octobre 2015 (2015-10-14), pages 61-73, XP029309117, ISSN: 0010-4825, DOI: 10.1016/J.COMPBIOMED.2015.10.002
- WENDA HE ET AL: "Mammographic Segmentation and Risk Classification Using a Novel Binary Model Based Bayes Classifier", 8 juillet 2012 (2012-07-08), BREAST IMAGING, SPRINGER BERLIN HEIDELBERG, BERLIN, HEIDELBERG, PAGE(S) 40 - 47, XP047013227, ISBN: 978-3-642-31270-0 section 2

## Description

### DOMAINE DE L'INVENTION

La présente invention concerne de manière générale un système informatique, une installation et un procédé pour le traitement d'image.

### ART ANTERIEUR

Dans le domaine de la radiographie médicale, et en particulier dans le domaine de la mammographie, un radiologue doit pouvoir interpréter l'image correspondant à la radiographie réalisée de manière fiable afin de déceler d'éventuelles anomalies dans l'objet radiographié.

L'image correspondant à une mammographie peut comprendre une ou des régions, dites régions d'intérêt, qui peuvent présenter des anomalies correspondant à des pathologies.

Ces régions doivent retenir l'attention du radiologue alors que d'autres régions, dites régions de non-intérêt ou polluantes, correspondent à des parties de l'objet radiographié, par exemple à des structures linéaires (vaisseaux, trames conjonctives) saines, qui n'ont en soit pas d'intérêt particulier pour le radiologue.

La présence de ces régions peut cependant gêner l'analyse de l'image par le radiologue, et peut en particulier perturber l'identification d'anomalies dans une région d'intérêt.

Il est connu de modifier les régions polluantes en appliquant aux différents points de la région polluante une même valeur d'intensité ou encore de rehausser l'intensité des régions d'intérêt, mais le résultat obtenu est souvent peu satisfaisant car un tel traitement de la région polluante peut également perturber l'analyse du radiologue et gêner ainsi son interprétation de la ou des régions d'intérêt.

On connait notamment du document "Breast image pre-processing for mammographie tissue segmentation", et du document US2016133033A1 des méthodes de traitement d'images qui proposent de modifier ou reconstruire une image acquise par mammographie. Cependant ces méthodes de modification ou de reconstruction peuvent également perturber l'interprétation l'analyse du radiologue.

La présente invention a pour but de proposer un nouveau système informatique, une nouvelle installation et un nouveau procédé permettant de pallier tout ou partie des problèmes exposés ci-dessus.

### RESUME DE L'INVENTION

A cet effet, l'invention a pour objet un procédé mis en œuvre à l'aide d'un système informatique pour le traitement d'au moins une partie, appelée région polluante, d'une image numérique d'un objet, selon la revendication 1.

Selon un aspect particulier, l'objet est un organe d'un corps humain ou animal.

On peut prévoir qu'une ou des parties, telles que des structures linéaires, d'une région polluante de l'image, en particulier lorsqu'il s'agit d'une image de sein, soit remplacée par une ou des parties d'image correspondant à des zones moins attirantes visuellement, telles que des zones de graisse.

Le procédé peut aussi comporter une ou plusieurs des caractéristiques suivantes prises dans toute combinaison techniquement admissible.

Selon une caractéristique avantageuse de l'invention, le procédé comprend aussi l'identification d'au moins un groupe de points de l'image correspondant à une région d'intérêt en fonction des valeurs d'intensité initiale I₀ des points de l'image et de ladite au moins une métadonnée.

Selon une caractéristique avantageuse de l'invention, l'étape d'identification d'au moins un groupe de points de l'image en tant que région polluante comprend la comparaison de ces points de l'image avec d'autres groupes de points de référence mémorisés dans une base de données, chaque groupe de points de référence étant associé à au moins une métadonnée, dite métadonnée de référence.

Le procédé comprend la fourniture d'un ensemble d'éléments texturants stockés dans une mémoire de données,
chaque élément texturant comprenant une pluralité de points et des valeurs d'intensité associées aux points,
et l'étape de remplacement des valeurs d'intensité initiale I₀ d'au moins une partie des points de ladite au moins une région polluante par de nouvelles valeurs d'intensité comprend :
   - la sélection d'un élément texturant parmi plusieurs éléments texturants ou la génération d'un nouvel élément texturant à partir d'un ou de plusieurs éléments texturants sélectionnés,
   - le remplacement des valeurs d'intensité initiale I₀ d'au moins une partie des points de ladite au moins une région polluante par les valeurs d'intensité d'au moins une partie des points de l'élément texturant sélectionné ou du nouvel élément texturant généré.

Selon un aspect particulier, chaque élément texturant comprend au moins une métadonnée et la sélection d'un ou plusieurs éléments texturants est réalisée en fonction de cette métadonnée.

Selon un aspect particulier, pour masquer un groupe de points de l'image identifié comme formant partie d'une région polluante, le procédé comprend la sélection ou la génération d'un élément texturant dont les caractéristiques (qui peuvent se présenter en tant que métadonnées, par exemple des données de tailles, de valeur ou moment d'intensité...), se rapprochent le plus des caractéristiques de la région polluante, à l'exception bien entendu des caractéristiques correspondant au type de tissu de la région polluante.

Le type de tissu peut être par exemple un vaisseau. Ainsi une région polluante de type vaisseau sain peut être remplacée par un élément texturant dont le type de tissu est de la graisse et dont les caractéristiques, autres que le type de tissu, sont proches de celles de la région polluante d'origine, de celles des régions voisines, et/ou de celles de l'image d'origine dans son intégralité.

L'élément texturant sélectionné ou le nouvel élément texturant généré présente une métadonnée correspondant à un type de tissu, par exemple de la graisse, qui est distinct du type de tissu, par exemple un vaisseau sanguin, de la région polluante à remplacer.

Selon une caractéristique avantageuse de l'invention, ladite au moins une métadonnée associée à l'image comprend une métadonnée définie par corrélation avec au moins une partie d'une autre image de l'objet.

Ladite autre image utilisée pour la corrélation peut être une image dudit objet mais acquise sous un autre angle ou à un autre temps. Ladite au moins une métadonnée peut être par exemple la localisation, ou le type région polluante ou d'intérêt, en particulier lorsque la corrélation se fait par comparaison des images prises à des temps différents.

Selon une caractéristique avantageuse de l'invention, pour une région polluante identifiée, le procédé comprend :
- le calcul d'au moins un moment mathématique à partir des valeurs d'intensité, tel que la valeur moyenne d'intensité I_{moy} ou la variance d'intensité, des points d'au moins une partie de ladite région polluante identifiée,
- la sélection d'un groupe de points, appelé élément texturant, parmi plusieurs éléments texturants prédéfinis, ou la génération d'un nouvel élément texturant à partir de plusieurs éléments texturants sélectionnés,
la sélection ou génération d'élément texturant étant réalisée en fonction dudit au moins un moment mathématique calculé, tel que ladite valeur moyenne d'intensité I_{moy} ou ladite variance d'intensité, des points de ladite au moins une partie de région polluante, les points dudit élément texturant sélectionné ou dudit élément texturant généré présentant un ensemble de valeurs d'intensité différent de celui des points de ladite au moins une partie de ladite région polluante identifiée ;
et au moins une partie des points dudit élément texturant sélectionné ou dudit nouvel élément texturant généré remplacent les points de ladite au moins une partie de la région polluante identifiée.

Selon un aspect particulier, les intensités initiales d'une zone de l'image (groupe de points), tels que les points d'une cellule de l'image, sont utilisées pour, par comparaison avec des échantillons d'image de référence, attribuer au moins une métadonnée aux points de cette zone, permettant de classifier le type de matière de cette zone, par exemple un type de tissu.

Puis cette métadonnée, éventuellement avec d'autres données, est utilisée pour sélectionner, parmi un ensemble d'éléments texturants prédéfinis, un élément texturant approprié prédéfini, ou générer un nouvel élément texturant à partir de plusieurs éléments texturants prédéfinis sélectionnés, afin de remplacer les points de la zone d'origine de l'image par les points de cet élément texturant.

La ou les métadonnées associées à une zone de l'image peuvent ainsi permettre d'identifier le type physiologique de la zone de l'image et de choisir au moins un élément texturant approprié parmi une pluralité d'éléments texturants précédemment mémorisés dans une base donnée. D'autres métadonnées de ladite zone de l'image d'origine peuvent être utilisées pour réaliser cette sélection, comme expliqué ci-après.

On peut prévoir que chaque élément texturant mémorisé soit lui aussi muni de métadonnées pour permettre d'analyser l'acceptabilité ou non de cet élément texturant vis-à-vis de ladite zone de l'image en vue du remplacement de ladite zone de l'image.

Les points qui composent cet élément texturant (et qui présentent un ensemble de valeurs d'intensité prédéfinies différent de celui des points de ladite zone de l'image initiale) sont utilisés pour remplacer les points de ladite zone de l'image initiale.

Le remplacement fait qu'il ne s'agit pas d'une opération mathématique de traitement d'intensité appliquée à l'image comme cela peut exister dans l'état de la technique, qui pourrait être réversible par une opération inverse, mais bien une substitution irréversible des points de ladite zone de l'image par d'autres points issus d'au moins un élément texturant extérieur (ou étranger) à l'image d'origine.

Selon un aspect particulier, il n'existe pas de transformation mathématique inverse permettant de retrouver les valeurs d'intensité des points d'origine à partir des intensités des points de l'image traitée qui ont remplacés lesdits points d'origine. L'utilisation d'un élément texturant pour remplacer la zone concernée se distingue donc d'un simple traitement des intensités de la zone, du fait qu'il s'agit d'un élément texturant disponible séparément de l'image et sélectionné parmi plusieurs éléments texturants, et éventuellement modifié/ajusté, ou d'un nouvel élément texturant généré à partir d'élément(s) texturant(s) sélectionné(s), et qui vient remplacer la zone concernée de l'image.

Ainsi à titre d'exemple et notamment dans le cas d'une image d'un sein, les points d'une zone de l'image peuvent présenter des valeurs d'intensité initiales qui permettent, éventuellement en combinaison avec d'autres données, d'identifier qu'il s'agit d'une partie d'un vaisseau sain. Cette identification peut être réalisée à l'aide d'un algorithme d'apprentissage statistique utilisant par exemple des caractéristiques de forme et/ou de déviation d'intensité pour conclure sur le type de tissu. Une métadonnée correspondant à un vaisseau sain peut alors être associé aux points de cette zone de l'image.

A titre d'exemple, l'élément texturant peut comprendre en tant que métadonnées la localisation dans l'objet de la matière qui correspond à cet élément texturant. Ainsi, lorsque l'élément texturant correspond à un tissu de type graisse, une métadonnée supplémentaire peut préciser qu'il s'agit d'une graisse sous-cutanée.

Selon un aspect particulier, la ou les régions d'intérêt ne sont pas remplacées par des éléments texturants

Cette métadonnée des points de la zone, éventuellement en combinaison avec d'autres données, permet ensuite de sélectionner un élément texturant approprié dont les points sont alors utilisés pour remplacer les points de la zone de l'image d'origine.

C'est ainsi qu'un élément texturant, auquel est associé une métadonnée représentative du fait que les caractéristiques de cet élément texturant correspondent à de la graisse, peut être sélectionné parmi plusieurs éléments texturants pour remplacer la zone de l'image d'origine correspondant à ladite partie de vaisseau sain, afin de ne pas attirer l'œil du radiologue sur cette zone de l'image, tout en gardant l'aspect visuel réaliste et familier du sein à l'œil du radiologue.

On comprend en effet que dans l'image, un vaisseau sain est une région qui n'a pas d'intérêt pour le radiologue et risquerait de polluer son analyse si elle restait présente telle quelle dans l'image. Une simple modification de l'image par exemple en réhaussant les valeurs d'intensité d'autres zones risquerait encore de gêner le radiologue. De manière similaire une simple normalisation des valeurs d'intensité ne serait pas suffisante. Le fait de remplacer cette zone de vaisseau par un élément texturant sélectionné sur la base des caractéristiques de cette zone de vaisseau permet de conserver une image naturelle du sein tout en évitant que le radiologue ait l'œil attiré par cette zone. Le radiologue peut ainsi se concentrer naturellement sur la ou les autres zones qui sont identifiées comme des zones d'intérêt.

On peut prévoir que les intensités des points de l'élément texturant sélectionné pour remplacer les points d'origine soient en outre modifiées en fonction de caractéristiques ou métadonnées (telles qu'un moment d'intensité, un paramètre de classification) de groupes de points voisins pour assurer une continuité ou une transition visuelle douce entre la zone remplacée et la ou les zones voisines.

Autrement dit, pour une zone correspondant à au moins une partie de région polluante, on sélectionne, ou on génère à partir d'éléments texturants sélectionnés, un élément texturant dont les caractéristiques, notamment d'intensité, permettent un remplacement des points de la zone par les points dudit élément texturant qui soit compatible avec un maintien de l'aspect réaliste de l'objet (par exemple un sein) de l'image ainsi modifiée, tout en réduisant l'attrait de l'œil de l'opérateur sur cette zone pour qu'il puisse de concentrer sur d'autres zones de réel intérêt.

Selon une caractéristique avantageuse de l'invention, ladite étape de remplacement, encore appelée opération de masquage, comprend la décomposition de l'image de l'objet en blocs de points adjacents, appelés cellules.

Selon un aspect particulier, lorsque chacun des points d'une cellule appartient à une région d'intérêt, lesdits points restent inchangés dans la nouvelle image générée.

Selon une caractéristique avantageuse de l'invention, lorsque les points d'une cellule appartiennent à une région polluante et que les points des cellules voisines appartiennent aussi à ladite région polluante, lesdits points de la cellule sont remplacés par les points d'un élément texturant choisi parmi plusieurs éléments texturants prédéfinis.

Selon un mode de réalisation particulier, la sélection dudit élément texturant parmi ladite pluralité d'éléments texturants prédéfinis est fonction des valeurs d'intensité desdits points de la cellule.

Selon une caractéristique avantageuse de l'invention, lorsque les points d'une cellule appartiennent à une région polluante et que des points d'au moins une cellule voisine appartiennent à une catégorie autre que ladite région polluante, par exemple à une région d'intérêt ou à une autre région polluante, un nouvel élément texturant est généré à partir d'au moins un, de préférence de plusieurs, éléments texturants prédéfinis, et en fonction des points de ladite au moins une cellule voisine, les points de ladite cellule étant remplacés par les points dudit nouvel élément texturant.

Selon une caractéristique avantageuse de l'invention, lorsqu'une cellule contient à la fois des points d'une région d'intérêt et d'une région polluante, la cellule est décomposée en sous-cellules, qui peuvent être elles-mêmes décomposées en sous-cellules jusqu'à ce chaque sous-cellule contienne uniquement des points de région d'intérêt ou uniquement des points de région polluante.

Selon une caractéristique avantageuse de l'invention, le procédé comprend une étape de traitement de l'image acquise dans laquelle la partie de l'image correspondant à l'objet, appelée image de l'objet, est isolée par rapport au fond de l'image acquise.

Selon une caractéristique avantageuse de l'invention, le procédé comprend une étape de détermination d'une région, dite région de contour, correspondant à une zone de contour de l'objet.

Selon une caractéristique avantageuse de l'invention, le procédé comprend l'identification, dans la partie d'image correspondant à l'objet, d'au moins une structure géométrique, par exemple une structure linéaire. Le procédé comprend l'attribution à ladite au moins une structure géométrique identifiée d'un paramètre de classification présentant une première valeur, correspondant à une catégorie d'intérêt, lorsqu'une corrélation est établie entre ladite structure géométrique et au moins une région d'intérêt ; et une deuxième valeur, correspondant à une catégorie de non-intérêt, lorsqu'il n'existe pas de corrélation entre ladite structure géométrique et la ou les régions d'intérêt.

Selon une caractéristique avantageuse de l'invention, l'objet étant comprimable sous l'effet d'une force de compression donnée, ladite au moins une métadonnée comprend l'épaisseur de l'objet à l'état comprimé et la force de compression appliquée à l'objet.

L'invention concerne également un produit de programme informatique non transitoire comprenant des instructions de code de programme pour l'exécution des étapes d'un procédé tel que proposé ci-dessus, lorsque ledit programme est exécuté par un processeur d'un système informatique.

L'invention concerne également un support d'enregistrement sur lequel est stocké le programme informatique.

L'invention concerne également un système informatique comprenant une mémoire contenant des instructions de code de programme pour l'exécution des étapes d'un procédé tel que proposé ci-dessus, un processeur permettant d'exécuter lesdites instructions de code de programme, une mémoire permettant de mémoriser des données d'image et des métadonnées, et de préférence un écran d'affichage.

L'invention concerne également une installation comprenant un système informatique tel que proposé ci-dessus, et un appareil à rayons X, ledit appareil comprenant un dispositif émetteur apte à émettre un faisceau de rayons X et un dispositif récepteur apte à recevoir les rayons X émis par le dispositif émetteur après leur passage à travers l'objet, et l'appareil à rayons X étant configuré pour transmettre au système informatique des données d'image, correspondant aux rayons X reçus par le dispositif récepteur après leur passage à travers l'objet, et, éventuellement, des métadonnées comprenant par exemple un ou des paramètres relatifs à la puissance ou à l'énergie des rayons X produits.

### BREVE DESCRIPTION DES DESSINS

D'autres caractéristiques et avantages de l'invention ressortiront encore de la description qui suit, laquelle est purement illustrative et non limitative et doit être lue en regard des dessins annexés pour lesquels :
- la figure 1 est une vue schématique d'un système informatique conformément à un mode de réalisation de l'invention ;
- la figure 2 est une vue schématique de l'image d'un sein acquise par un appareil à rayons X selon un mode de réalisation de l'invention ;
- la figure 3 est une vue schématique de l'image de la figure 2 après suppression du fond de l'image selon un mode de réalisation de l'invention ;
- la figure 4 est une vue schématique de l'image de la figure 3 pour laquelle une zone déterminée de contour du sein est schématisée, conformément à un mode de réalisation de l'invention ;
- la figure 5 est une vue schématique de l'image de la figure 3 dans laquelle sont schématisées des régions polluantes conformément à un mode de réalisation de l'invention ;
- la figure 6 est une vue schématique de l'image de la figure 3 dans laquelle est schématisée une région d'intérêt conformément à un mode de réalisation de l'invention ;
- la figure 7 est une vue schématique de l'image de la figure 3 dans laquelle est schématisée une structure géométrique associée à une région d'intérêt conformément à un mode de réalisation de l'invention ;
- la figure 8 est une vue schématique de l'image de la figure 3 dans laquelle est schématisée une structure géométrique associée à une ou des régions polluantes conformément à un mode de réalisation de l'invention ;
- la figure 9 est une vue schématique de l'image de la figure 3 dans laquelle sont schématisées les régions polluantes, la région d'intérêt, et la zone de contour et les structures géométriques schématisées dans les figures 4 à 8 conformément à un mode de réalisation de l'invention ;
- la figure 10 est une vue schématique de l'image de la figure 9 à laquelle est appliquée une grille pour décomposer l'image en cellules conformément à un mode de réalisation de l'invention ;
- la figure 11 est une vue d'une partie de l'image de la figure 10 montrant une cellule C1 d'une région d'intérêt et des cellules voisines conformément à un mode de réalisation de l'invention ;
- la figure 12 est une vue d'une partie de l'image de la figure 10 montrant une cellule C2 d'une première région polluante et des cellules voisines conformément à un mode de réalisation de l'invention ;
- la figure 12A est une vue schématique montrant plusieurs éléments texturants conformément à un mode de réalisation de l'invention ;
- la figure 12B est une vue schématique de la partie d'image de la figure 12 après modification de la cellule C2 en fonction d'au moins un élément texturant de la figure 12A conformément à un mode de réalisation de l'invention ;
- la figure 13 est une vue d'une partie de l'image de la figure 10 montrant une cellule C3 d'une deuxième région polluante et des cellules voisines ;
- la figure 13A est une vue schématique montrant trois éléments texturants conformément à un mode de réalisation de l'invention ;
- la figure 13B est une vue schématique de la partie d'image de la figure 13 après modification de la cellule C3 en fonction d'au moins un élément texturant de la figure 13A conformément à un mode de réalisation de l'invention ;
- la figure 14 est une vue d'une partie de l'image de la figure 10 montrant une cellule C4 d'une région polluante et des cellules voisines conformément à un mode de réalisation de l'invention ;
- la figure 14A est une vue schématique montrant trois éléments texturants dont un (celui du bas) a été généré en fonction des deux autres conformément à un mode de réalisation de l'invention ;
- la figure 14B est une vue schématique de la partie d'image de la figure 14 après modification de la cellule C4 en fonction de l'élément texturant généré de la figure 14A conformément à un mode de réalisation de l'invention ;
- la figure 15 est une vue d'une partie de l'image de la figure 10 montrant une cellule C5 comprenant des points de région d'intérêt et de région polluante conformément à un mode de réalisation de l'invention ;
- la figure 15A est vue de la figure 15 après application d'un maillage plus fin à la cellule C5 pour que chaque sous-cellule comprenne uniquement des points de région d'intérêt ou uniquement des points de région polluante conformément à un mode de réalisation de l'invention ;
- la figure 16 est une vue d'une image générée à partir de l'image de la figure 10 après modification de régions polluantes à l'aide d'éléments texturants conformément à un mode de réalisation de l'invention.

### DESCRIPTION DETAILLEE

Le concept de l'invention est décrit plus complètement ci-après avec référence aux dessins joints, sur lesquels des modes de réalisation du concept de l'invention sont montrés. Sur les dessins, la taille et les tailles relatives des éléments peuvent être exagérées à des fins de clarté. Des numéros similaires font référence à des éléments similaires sur tous les dessins. Cependant, ce concept de l'invention peut être mis en œuvre sous de nombreuses formes différentes et ne devrait pas être interprété comme étant limité aux modes de réalisation exposés ici. Au lieu de cela, ces modes de réalisation sont proposés de sorte que cette description soit complète, et communiquent l'étendue du concept de l'invention aux hommes du métier. Les modes de réalisation qui suivent sont examinés, par souci de simplification, en relation avec la terminologie et la structure d'un appareil de radiographie et d'un système de traitement d'image, en particulier dans le contexte d'une mammographie.

Une référence dans toute la spécification à « un mode de réalisation » signifie qu'une fonctionnalité, une structure, ou une caractéristique particulière décrite en relation avec un mode de réalisation est incluse dans au moins un mode de réalisation de la présente invention. Ainsi, l'apparition de l'expression « dans un mode de réalisation » à divers emplacements dans toute la spécification ne fait pas nécessairement référence au même mode de réalisation. En outre, les fonctionnalités, les structures, ou les caractéristiques particulières peuvent être combinées de n'importe quelle manière appropriée dans un ou plusieurs modes de réalisation.

Le traitement d'image décrit ci-après vise le masquage de certaines zones d'une image générée par une acquisition de rayons X en 2D ou 3D suite à l'exposition d'un objet, tel qu'un sein, à ces rayons X. Le masquage est réalisé de manière à garder l'aspect naturel de l'objet visualisé. Le masquage peut être réalisé de manière irréversible en remplaçant certains ensembles de points de l'image de l'objet par d'autres ensembles de points, ci-après appelés éléments texturants, qui sont définis et mémorisés indépendamment de l'image de l'objet. On peut ainsi prévoir que ces éléments texturant soient présents en mémoire, antérieurement à l'acquisition de l'image de l'objet. Autrement dit, un élément texturant utilisé pour remplacer une zone donnée (considérée comme une région polluante) de l'image n'est initialement pas généré à partir du contenu de la zone à remplacer, ni du contenu d'une autre zone de l'image d'origine. On peut en particulier prévoir que les éléments texturant soient définis à partir de zones d'autres images considérées comme des images de référence.

Bien entendu, et comme expliqué ci-après, si les éléments texturant ne sont pas définis en fonction des caractéristiques des zones de l'image à remplacer, la sélection ou la génération d'un élément texturant parmi l'ensemble d'éléments texturant s'effectue pour autant en tenant compte des caractéristiques de la zone d'image à remplacer. Par ailleurs, après sélection ou génération de cet élément texturant, celui-ci peut être éventuellement modifié pour être adapté en fonction de la ou des zones voisines de la zone à remplacer, en particulier pour offrir une continuité visuelle entre la zone remplacée et son environnement.

Le traitement est en particulier applicable sur les images radiographiques 2D et 3D d'éléments élastiques et déformables. En variante, ledit objet peut être rigide ou déformable, tel qu'un poumon, ou plus généralement, une cage thoracique, mais non soumis à un effort de compression lors de l'acquisition d'image.

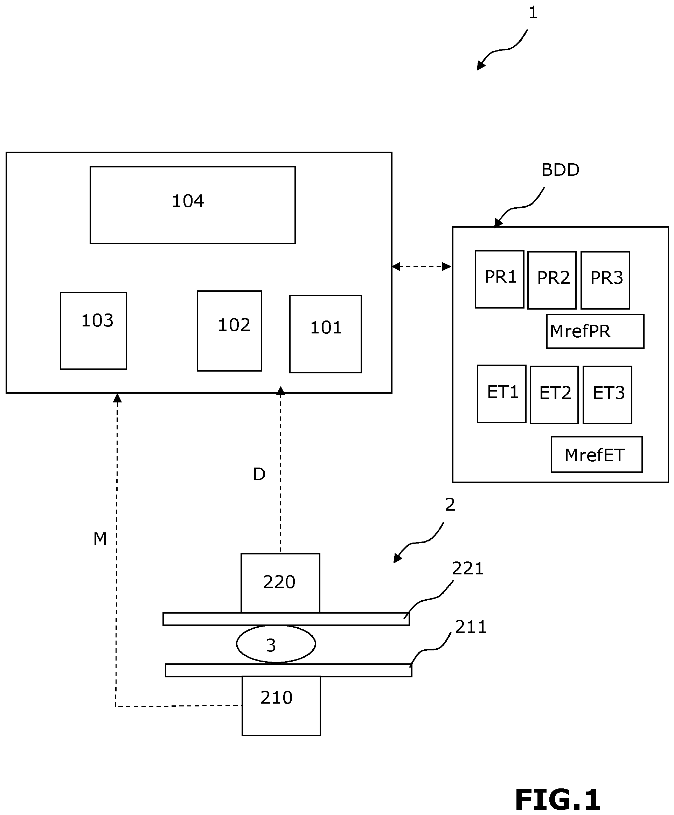

A la figure 1 est illustré un système informatique 1 et un appareil 2 de radiographie à rayons X.

Comme détaillé ci-après, le système informatique 1 permet le traitement d'une ou plusieurs régions, dites régions polluantes, d'une image numérique d'un objet. Dans la suite de la description ledit objet 3 est un sein et l'image obtenue à l'aide de l'appareil 2 de radiographie est une mammographie. Bien entendu l'invention est applicable à d'autre objets élastiquement comprimables. En variante, comme rappelé ci-dessus, ledit objet peut être rigide ou déformable mais non soumis à un effort de compression lors de l'acquisition d'image.

Une région polluante est dite "polluante" au sens où elle ne présente pas en soi d'intérêt vis-à-vis de l'analyse de l'image que doit effectuer le radiologue, contrairement à d'autres régions, dites région d'intérêt, qui elles présentent un intérêt pour le radiologue, afin par exemple d'identifier une anomalie.

Une ou des régions polluantes de l'image peuvent perturber l'analyse par le radiologue d'une ou des régions d'intérêt en l'empêchant de concentrer son analyse sur la ou les régions d'intérêt.

Comme détaillé ci-après, l'invention permet de conserver la ou chaque région d'intérêt et de limiter le risque que l'analyse du radiologue soit perturbée par la présence d'une ou plusieurs régions polluantes, tout en conservant les caractéristiques générales graphiques du sein (telles que sa forme, sa texture) afin de ne pas non plus perturber l'opérateur par un traitement graphique qui serait inadapté de la ou des régions polluantes, comme cela pourrait être le cas si les points de la ou les régions polluantes étaient simplement affectés d'une même valeur d'intensité ou si les points de la ou les régions polluantes subissaient une opération mathématique sur leur valeurs d'intensités de sorte que la région modifiée conserverait ses caractéristiques graphiques polluantes. Par exemple, une région qui présenterait graphiquement un tissu correspondant à un vaisseau sain d'un sein et qui subirait des opérations mathématiques réversibles sur ces valeurs d'intensité conserverait l'aspect graphique général de ce vaisseau, ce qui risquerait encore de perturber l'analyse de l'image de l'objet. La solution selon l'invention qui prévoit un masquage par remplacement de la région polluante par un élément texturant défini initialement indépendamment de cette région permet dans l'exemple ci-dessus d'une région polluante avec un vaisseau sain d'un sein de sélectionner un élément texturant correspondant à de la graisse mammaire et de substituer, éventuellement après modification de cet élément texturant, les points de la région polluante par les points de l'élément texturant, de manière à faire apparaitre visuellement de la graisse mammaire en lieu et place du vaisseau sain. On comprend que pour une image de sein la graisse mammaire est un type de tissu qui ne va pas attirer l'œil du professionnel qui analyse l'image, à la différence d'un vaisseau, de sorte que le professionnel peut concentrer son analyse sur une ou des régions d'intérêt qui ne subissent pas à un tel masquage, c'est-à-dire qui ne sont pas remplacées par des éléments texturants.

### • Acquisition de l'image

L'appareil 2 de radiographie est utilisé pour acquérir une image du sein 3 en exposant le sein 3 à des rayons X (ou faisceaux de rayons X). En particulier, l'appareil 2 comprend des plaques 211, 221 de compression entre lesquelles est positionné le sein 3. Le sein 3 est alors comprimé entre les plaques 211, 221 de compression sous l'effet d'une force de compression donnée, de préférence réglable.

Un dispositif 210 générateur de rayons X génère alors des rayons X auxquels le sein 3 est exposé. Le dispositif 210 générateur peut à cet effet comprendre un tube d'émission de rayons X et un système électronique permettant de régler l'énergie ou puissance, par exemple la tension et l'intensité électrique, utilisée pour produire les rayons X.

Un dispositif 220 récepteur reçoit les rayons X après leur passage à travers le sein 3. Le dispositif 220 récepteur peut également recevoir des rayons qui passent autour de l'objet sans le traverser et qui définissent alors le fond de l'image. Les rayon X reçus par le dispositif récepteur 2 présentent différentes valeurs d'intensité en fonction des caractéristiques des différentes zones du sein traversées et permettent ainsi de former une image IMG1 correspondant à la mammographie.

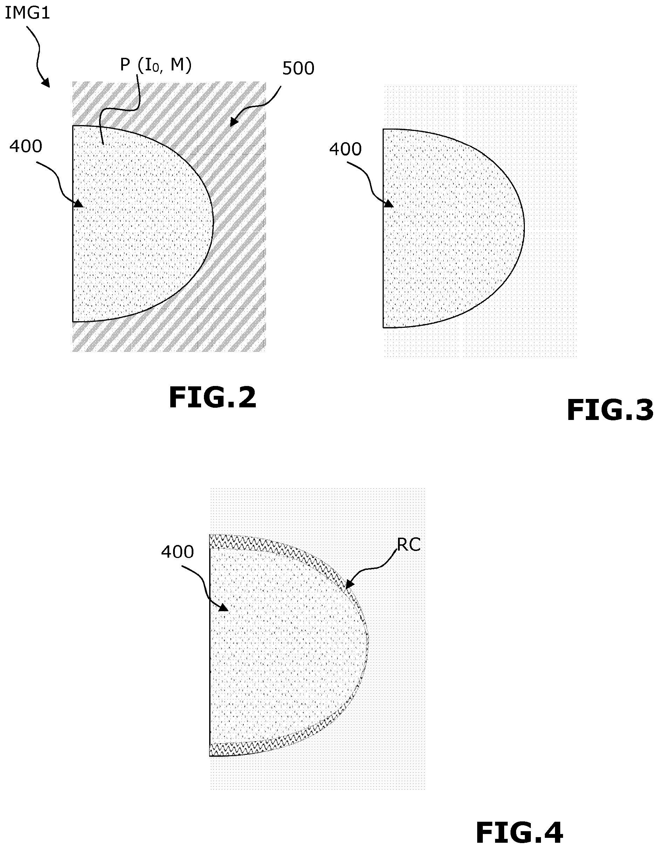

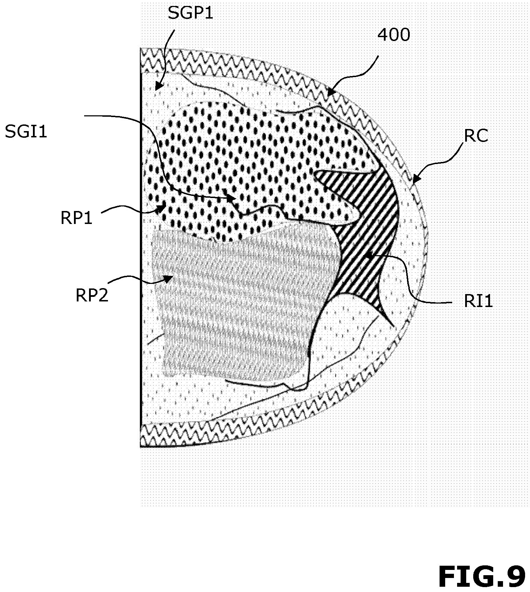

Les points de l'image obtenue IMG1 correspondent à des points du sein 3 qui ont été exposés aux rayons X ou à des points de l'environnement du sein 3 formant le fond de l'image. Il est à noter que les images ou portions d'images représentées aux figures 2 à 16 sont des schémas simplifiés pour faciliter la compréhension du procédé de traitement.

Sur le plan informatique l'image est représentée par un ensemble de points définis dans l'espace. L'image peut être caractérisée par tout ou partie de ses dimensions, telles que la largeur, la hauteur et la profondeur.

Chaque point est caractérisé par des coordonnées, par exemple 2D (i.e. image planaire), 3D (ex. volume ou 2D + temps), 4D (ex. volume 3D + temps), et une valeur numérique associée à chaque point correspondant à une valeur d'intensité lumineuse. Les valeurs numériques peuvent être bornées. Les valeurs numériques peuvent en particulier appartenir à un sous-ensemble de l'espace des chiffres naturels ou réels.

Plusieurs types d'acquisition d'image sont ainsi possibles. On peut distinguer à titre d'exemple les acquisitions suivantes :
- une image 2D ;
- plusieurs images 2D acquises suivant des incidences différentes où l'angle d'exposition est connu, ou suivant des incidences différentes où l'angle n'est pas connu ;
- une image 3D.

Pour chaque type d'acquisition, de nombreux paramètres d'acquisition (i.e. métadonnées) peuvent être fournis. Par exemple, tout ou parties des paramètres suivants :
- le fabriquant et/ou le modèle de l'appareil d'acquisition ;
- les caractéristiques d'émission des rayons X, telles que puissance, durée...
- le oules angles d'incidence de l'acquisition ;
- l'épaisseur de l'objet étudié, de préférence sous compression.

Tout ou partie de ces paramètres peut être utilisé(e) comme métadonnées associées à une image acquise.

Dans la suite de la description, il est fait référence à une image, mais bien entendu la description s'applique aussi à une pluralité d'images. En particulier, comme rappelé ci-dessus, on peut prévoir que plusieurs images de l'objet soient acquises suivant différents angles d'exposition de l'objet aux rayons X, c'est-à-dire différents angles d'incidence des rayons par rapport à l'objet.

Le système informatique comprend une mémoire 103 de données dans laquelle sont mémorisées des données D de l'image acquise à l'aide du dispositif récepteur 220. Les données D peuvent ainsi comprendre pour chaque point P de l'image, des coordonnées dudit point et une valeur d'intensité I₀, dite valeur d'intensité initiale.

Le système informatique 1 comprend aussi une mémoire 101 contenant des instructions de code de programme (instructions informatiques) pour l'exécution des étapes d'un procédé comprenant des étapes telles que décrites ci-après. Un processeur 102 permet d'exécuter lesdites instructions de code de programme.

Comme rappelé ci-dessus, la mémoire 103 permet de mémoriser les données d'image D acquises à l'aide du dispositif 220 récepteur. Des métadonnées M présentées ci-dessous sont également mémorisées dans la mémoire 103 ou dans une autre mémoire. Le système informatique peut aussi comprendre un écran d'affichage 104 pour l'affichage de l'image.

L'intensité initiale I₀ d'un point P de l'image associé à un point du sein 3, correspond à la quantité de photons qui ont frappé un point correspondant du sein 3. Par ailleurs, comme évoqué ci-dessus, chaque point P de l'image est associé à des métadonnées M. Ainsi comme illustré de manière schématique à la figure 2, l'image acquise IMG1 présente des points P auxquels sont associés des coordonnées, une valeur d'intensité initiale Io et des métadonnées M. Une partie 400 de l'image IMG1 correspond à l'objet 3 et une autre partie 500 correspond au fond de l'image.

Selon un mode de réalisation préféré, les métadonnées M comprennent :
- l'épaisseur du sein 3 à l'état comprimé et la force de compression appliquée à l'objet,
- les valeurs de tension et d'intensité électrique utilisées pour produire les rayons X.

Comme expliqué ci-avant, d'autres métadonnées ou des métadonnées supplémentaires peuvent être rajoutées, ou certaines métadonnées peuvent être remplacées par des métadonnées équivalentes. Par exemple, les valeurs de tension et d'intensité électrique peuvent être remplacées par une grandeur représentative de la puissance ou de l'énergie utilisée pour générer les rayons X. De même en l'absence des plaques de compression 221 et 222, l'épaisseur de l'objet comprimé peut ne pas être renseignée.

Les métadonnées peuvent être en tout ou partie fournies par l'appareil 2 en le connectant au système informatique 1, ou peuvent être calculées à partir de données initiales. Les métadonnées peuvent également être entrées par un utilisateur dans le système 1 informatique à l'aide d'une interface de saisie de données, telle qu'un clavier ou un écran tactile. Les métadonnées peuvent comprendre des métadonnées dite primaires fournies lors de l'acquisition de l'image et des métadonnées dites secondaires calculées à partir de données primaires, telle que les valeurs d'intensité initiale et les métadonnées primaires.

Les métadonnées peuvent aussi comprendre la densité du sein 3. La densité peut servir à définir des sous-catégories parmi une catégorie donnée associée à une région de l'image. On peut prévoir que la densité soit une valeur calculée à partir des données initiales, i.e. une valeur calculée à partir des valeurs d'intensités et des métadonnées attribuées initialement à l'image acquise.

La partie de l'image qui correspond au sein 3 est appelée par la suite image 400 du sein (voir figure 2).

Selon un aspect particulier et comme illustré de manière schématique à la figure 3, la partie 400 de l'image du sein, est isolée par rapport au fond 500 de l'image acquise. Le traitement peut ainsi être ciblé sur la partie 400 de l'image correspondant au sein 3.

### • Région(s) polluante(s) et région(s) d'intérêt

Les valeurs d'intensité initiale I₀ des points P de l'image et lesdites métadonnées M de l'image sont utilisées pour identifier la ou les régions polluantes et la ou les régions d'intérêt.

Suite à l'identification d'une région polluante, un paramètre d'une valeur qui correspond à une catégorie de non-intérêt, et éventuellement une sous-catégorie, est alors attribué aux points de ladite région polluante. De manière similaire, suite à l'identification d'une région d'intérêt, un paramètre d'une valeur correspondant à une catégorie d'intérêt, et éventuellement une sous-catégorie, est alors attribué aux points de ladite région d'intérêt.

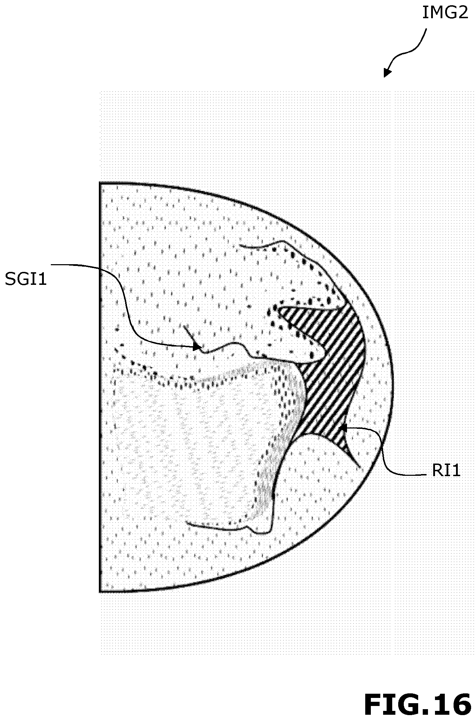

Après un traitement des points de l'image, expliqué ci-après, une nouvelle image IMG2 va être générée par remplacement des valeurs d'intensité initiale I₀ des points des régions polluantes par de nouvelles valeurs d'intensité (voir Figure 16).

Selon un aspect particulier, le procédé de remplacement, encore appelé opération de masquage, correspond au remplacement des valeurs d'intensité des points des zones de non-intérêt (encore appelées régions polluantes) par des valeurs d'intensité d'échantillons d'image. Ces échantillons d'image peuvent être des éléments texturants présentés ci-après, qui peuvent être choisis parmi des éléments texturants prédéfinis, ou générés à partir d'autres éléments texturants prédéfinis. Les éléments texturants, bien qu'initialement indépendant de l'image acquise, peuvent être en outre adaptés en fonction de caractéristiques de l'image ou de parties de l'image, par exemple de points de région(s) voisine(s) (qui peuvent être de type polluante ou d'intérêt) afin d'assurer une cohérence visuelle.

### • Identification de région(s) polluante(s) et de région(s) d'intérêt

Afin d'identifier si un groupe de points de l'image (région de l'image) correspond à une région polluante ou à une région d'intérêt, le groupe de points de l'image peut être comparé avec d'autres groupes de points de référence, par exemple les groupes de points PR1, PR2, PR3, mémorisés dans une base de données BDD. Ces groupes de points PR1, PR2, PR3 peuvent correspondre à des échantillons d'images.

Chaque groupe de point de référence PR1, PR2, PR3 mémorisé dans la base de données BDD comprend des valeurs d'intensité de référence et des métadonnées de référence MrefPR, et est associé à une catégorie correspondant au type région polluante ou au type région d'intérêt, et éventuellement à des sous-catégories, par exemple une sous-catégorie correspondant à une région de faible intensité ou une région de haute intensité. Un autre exemple peut être une sous-catégorie, par exemple une sous-catégorie de région d'intérêt, correspondant à une répartition des points décrivant une forme particulière, par exemple une forme en étoile. Avantageusement, les mêmes métadonnées sont associées aux différents points appartenant à un même groupe de points, et chacun des points présente une valeur d'intensité qui peut être différente de celles des autres points.

Les métadonnées de référence MrefPR des points de référence PR1, PR2, PR3 comprennent de préférence des métadonnées de même type que les métadonnées associées à l'image acquise IMG1. Les métadonnées de référence MrefPR peuvent ainsi comprendre de manière similaire aux métadonnées associées à l'image acquise :
- une valeur d'épaisseur de sein de référence à l'état comprimé et une valeur de force de compression correspondante,
- une ou des valeurs de paramètre(s) relatif(s) à la puissance ou énergie de rayons X, telles que des valeurs de tension et d'intensité électrique.

En particulier, pour un groupe de points donné d'une région, qui comme détaillé ci-après peut correspondre à une cellule de l'image, le système identifie un groupe de points de référence parmi les groupes PR1, PR2, ou PR3 mémorisés dans la base de données BDD, dont les métadonnées et les intensités des points se rapprochent le plus des métadonnées et des intensités initiales des points dudit groupe de points dont on souhaite identifier la catégorie.

Une fois identifié un groupe de points de référence dont les caractéristiques se rapprochent le plus des caractéristiques dudit groupe de points à catégoriser, on affecte audit groupe de points à catégoriser la catégorie (région polluante ou région d'intérêt), et éventuellement la ou chaque sous-catégorie, du groupe de points de référence identifié.

On peut prévoir qu'un groupe de points à catégoriser (ou la région correspondante) hérite de tout ou partie des métadonnées, et/ou des paramètres de catégorie, et éventuellement sous-catégorie, du groupe de points de référence correspondant identifié.

L'identification d'un groupe de points de référence qui se rapproche le plus d'un groupe de points donné de l'image peut s'effectuer à l'aide d'une fonction de corrélation.

A titre d'exemple, pour un groupe de points GP donné de l'image présentant des métadonnées M_{GP} et un moment statistique d'intensité S calculé à partir des valeurs d'intensité des points dudit groupe, le système calcule pour le groupe de points GP un score à l'aide d'une fonction d'évaluation V(GP) = f(GP, MGP, S)

Un score V_PR_1, V_PR_2, V_PR_3 est également calculé pour chaque région de référence à l'aide de cette fonction d'évaluation. Le système évalue alors une fonction objectif ou fonction de coût L (GP, PR_i) = f (V(GP), V_PR_i) pour chaque région de référence. Sera retenu le groupe de points de référence PR1, PR2, PR3 qui donne la valeur maximale ou minimale de la fonction objectif ou de la fonction de coût.

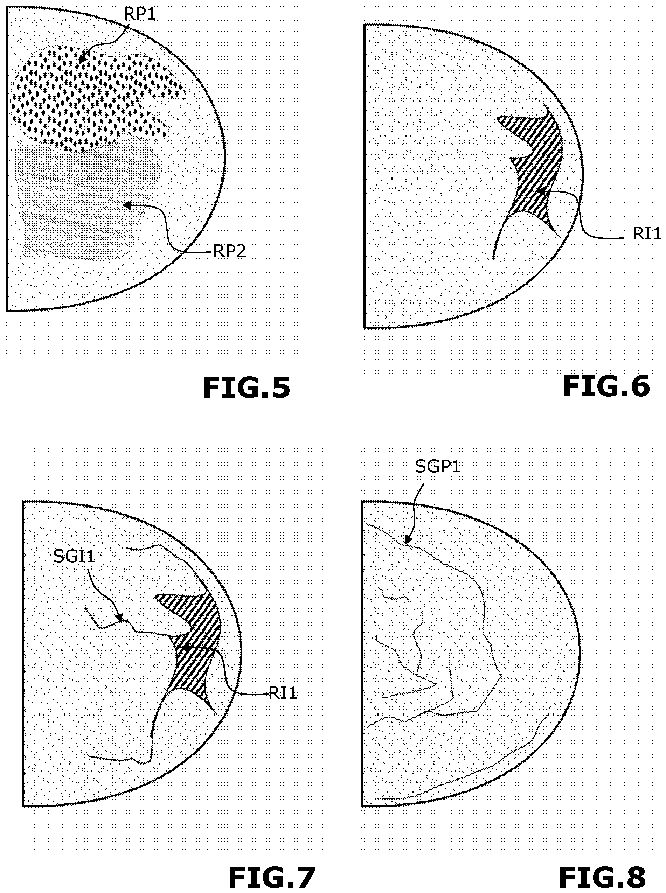

Dans l'exemple illustré aux figures, les régions polluantes (c'est-à-dire de non-intérêt) identifiées sont les régions RP1, RP2 (voir figure 5), et la région d'intérêt identifiée est la région RI1 (voir figure 6). On comprend que le procédé présenté s'applique également à d'autre configurations d'image présentant une ou plusieurs régions polluantes et une ou plusieurs régions d'intérêt.

### • Région de contour

Selon un aspect particulier et comme illustré à la figure 4, une région de contour RC de l'image 400 du sein 3, correspondant à une zone de contour du sein 3 peut être déterminée.

Dans l'exemple de la figure 4, les portions de plus grande largeur de la région de contour RC correspondent à des parties du sein en contact direct avec les plaques 211, 221 de compression.

La détermination d'une région de contour RC peut être réalisée par correspondance de forme, et éventuellement en fonction des valeurs d'intensité de la zone de contour et de la valeur de force appliquée au sein 3.

### • Identification de structures géométriques à géométrie ou forme particulière

Le système permet d'identifier dans l'image 400 du sein une ou des structures géométriques SGI1, SGP1. Une structure géométrique comprend par exemple une structure linéaire.

Ainsi, dans une première étape, une structure géométrique peut être identifiée par sa géométrie, par exemple une géométrie de ligne, brisée ou non. Ensuite, le système recherche une corrélation de la structure géométrique avec une ou des régions d'intérêt ou une ou des régions polluantes (non-intérêt). La corrélation peut être fonction des valeurs d'intensité lumineuse, et éventuellement des métadonnées. La fonction de corrélation peut être similaire à celle présentée ci-dessus.

Le système permet à l'aide d'une fonction de corrélation (par exemple similaire à celle présentée ci-dessus) d'attribuer à une structure géométrique identifiée un paramètre de classification présentant une première valeur, correspondant à une catégorie d'intérêt, lorsqu'une corrélation est établie entre ladite structure géométrique et au moins une région d'intérêt. Inversement, une deuxième valeur, correspondant à une catégorie de non-intérêt (ou polluante), est attribuée à la structure géométrique lorsqu'il n'existe pas de corrélation (suffisante) entre ladite structure géométrique et la ou les régions d'intérêt.

Ainsi comme illustré à la figure 7, le système identifie la structure géométrique SGI1 pour laquelle une corrélation est établie avec la région d'intérêt RI1 de sorte qu'un paramètre correspondant à la catégorie d'intérêt est attribué à ladite structure géométrique SGI1. Comme illustré à la figure 8, le système identifie aussi la structure géométrique SGP1 pour laquelle une corrélation est établie avec la région polluante RP1 de sorte qu'un paramètre correspondant à une catégorie de non-intérêt est attribué à ladite structure géométrique SGP1. On peut prévoir que la structure géométrique hérite de tout ou partie des métadonnées, et/ou des paramètres de catégorie, et éventuellement sous-catégorie, d'une région à laquelle elle est corrélée.

### • Eléments texturants

Pour réaliser le masquage, des éléments texturants ET1, ET2, ET3 collectés ou définis préalablement sont utilisés. Un élément texturant est une image à rayons X illustrant une partie d'objet de même nature que l'objet, ici le sein 3, dont l'image a été acquise.

Ledit élément texturant correspond par exemple à l'image d'une zone de graisse qui est moins attirante visuellement et donc moins perturbante qu'une zone de région polluante. Ainsi lorsque ces éléments texturants, éventuellement après modification, vont être utilisés pour remplacer des régions polluantes de l'image, afin de permettre au radiologue de se concentrer sur la ou les régions d'intérêt, le sein conservera néanmoins ses caractéristiques générales (forme et de texture) de sorte que le risque que ces remplacements perturbent le radiologue sera limité.

Les éléments texturants peuvent être stockés dans une base de données (locale ou distante) accessible lors de l'analyse, par exemple la base de données BDD.

Chaque élément texturant ET1, ET2, ET3 comprend un groupe de points et des métadonnées.

De manière similaire aux groupes de points de référence PR1, PR2, PR3, chaque élément texturant ET1, ET2, ET3 comprend des valeurs d'intensité et des métadonnées de référence MrefET, et est éventuellement associé à une sous-catégorie de région polluante par exemple une sous-catégorie correspondant à une région de faible intensité ou une région de haute intensité.

Avantageusement, les mêmes métadonnées sont associées aux différents points appartenant à un même groupe de points d'élément texturant, et chacun des points présente une valeur d'intensité qui peut être différente de celles des autres points.

Les métadonnées de référence MrefET des éléments texturants ET1, ET2, ET3 comprennent de préférence des métadonnées de même type que les métadonnées associées à l'image acquise IMG1. Les métadonnées de référence MrefET peuvent ainsi comprendre de manière similaire aux métadonnées associées à l'image acquise :
- une valeur d'épaisseur de sein de référence à l'état comprimé et une valeur de force de compression correspondante,
- une ou des valeurs de paramètre(s) relatif(s) à la puissance ou l'énergie de rayons X, telles que des valeurs de tension et d'intensité électrique.

A chaque élément texturant peuvent aussi être associées différentes données, telles que la taille de l'élément texturant, le type du tissu, des caractéristiques statistiques d'intensité. Ces données peuvent être également associées aux points de référence présentés ci-dessus.

Selon un mode de réalisation particulier, la variance (i.e. deuxième moment mathématique) d'intensité d'un ou de chaque groupe de points de référence est plus élevée que celle d'un ou de chaque élément texturant. Autrement dit, un élément texturant peut présenter une plus grande homogénéité d'intensité qu'un groupe de points de référence.

Selon un mode de réalisation particulier, la moyenne des valeurs d'intensité (i.e. premier moment mathématique) des points d'un ou de chaque groupe de points de référence est supérieure à celle d'un ou de chaque élément texturant.

On comprend que les éléments texturants sont distincts des groupes de points de référence qui servent à l'identification des régions polluantes ou d'intérêt.

Suivant un mode de réalisation préféré, l'opération de masquage a pour objectif de rendre invisible à l'opérateur une ou des régions dites polluantes et de les remplacer par des régions permettant d'atténuer l'attention lors de l'analyse d'image. Ainsi, les éléments texturants sont sélectionnés et classés par leurs propriétés d'intensité, notamment les valeurs d'intensité des points d'élément et des moments mathématiques associés. En outre la distribution statistique des points d'un élément texturant peut être prise en compte afin de déterminer le type de tissu représenté par ledit élément texturant.

Un masquage d'une région polluante peut être effectué par application à tout ou partie de ladite région polluante d'un masque composé à partir d'un ou plusieurs éléments texturants.

### • Sélection ou génération d'éléments texturants

Pour une région polluante RP1, RP2 identifiée, au moins un moment mathématique des valeurs d'intensité des points de ladite région, tel que la valeur moyenne d'intensité I_{moy} ou la variance d'intensité, est calculé. Différents moments mathématiques peuvent être calculés et pris seuls ou en combinaison pour l'identification de région polluante. Similairement un ou des moments mathématiques peuvent être calculés pour les cellules dans le voisinage de ladite région polluante.

Des éléments texturants ET121 ; ET133 sont sélectionnés parmi plusieurs éléments texturants ET121, ET122, ET123 ; ET131, ET132, ET133 prédéfinis, en fonction de caractéristiques, telles qu'un ou des moments mathématiques calculés de la région polluante et de préférence d'une ou plusieurs métadonnées associées, et éventuellement en fonction des moments mathématiques du voisinage de ladite région.

En particulier, pour un groupe de points donné d'une région, qui comme expliqué ci-après peut correspondre à une cellule de l'image, suite à l'application d'une grille sur l'image, on peut prévoir que le système sélectionne un élément texturant parmi les éléments texturants prédéfinis dans la base de données BDD, dont les métadonnées et les intensités des points se rapprochent le plus des métadonnées et des intensités initiales des points de la région polluante et éventuellement de son voisinage. La corrélation entre les éléments texturants et la région polluante peut être établie par une analyse d'une fonction objectif ou d'une fonction de coût prenant en compte une partie ou toutes les métadonnées et des intensités initiales de ladite région. A cet effet il est possible d'utiliser des fonctions de corrélation et d'objectif ou coût qui peuvent être similaires à celles présentées ci-avant.

On peut également prévoir qu'un nouvel élément texturant soit généré en fonction de plusieurs éléments texturants identifiés par exemple en fonction des moments mathématiques d'intensité correspondants.

Une fois l'élément texturant sélectionné ou généré à partir d'éléments texturants sélectionnés, le système utilise cet élément texturant comme masque de modification d'un groupe de points de la région polluante. En particulier, le système peut modifier les valeurs d'intensité des points de ce groupe en fonction des valeurs d'intensité des points dudit élément texturant. Cette modification peut être un remplacement des valeurs d'intensité des points de ce groupe par les valeurs d'intensité des points dudit élément texturant. Les intensités des autres groupes de points de la région peuvent aussi être modifiées en fonction, ou remplacées par, des intensités des groupes de points de l'élément texturant.

Les caractéristiques de l'élément texturant utilisé comme masque peuvent être adaptées (c'est-à-dire modifiées), par exemple en fonction d'une ou des zones voisines de la ou de chaque zone où est appliquée le masque pour ne pas s'écarter des caractéristiques générales (Intensité, texture) de l'image acquise.

Selon un mode de réalisation préféré, le masquage des régions de non-intérêt, dites "polluantes", est destiné à remplacer lesdites régions par un masque construit à partir des éléments texturants ou des compositions desdits éléments texturants.

Dans un mode de réalisation préféré, les éléments texturants constituant le masque présentent des caractéristiques d'intensité particulières.

A titre d'exemple, on peut prévoir que la valeur d'intensité minimale d'image corresponde à de l'air.

Les éléments texturants peuvent être sélectionnés ou générés à partir d'éléments texturants sélectionnés de manière à minimiser la moyenne d'intensité (i.e. premier moment mathématique) en se rapprochant d'un seuil prédéfini, noté Mₘᵢₙ, différent du minimum absolu (ex. intensité de l'air), et de manière à minimiser l'écart-type des valeurs d'intensité (c'est-à-dire pour maximiser l'homogénéité) en se rapprochant d'un seuil différent de zéro, noté Sₘᵢₙ. Selon un tel mode de réalisation, seuls les éléments texturants sont soumis à des contraintes sur un ou plusieurs de leurs moments mathématiques.

Selon un mode de réalisation particulier, un groupe de points de référence PR_i peut présenter les mêmes caractéristiques statistiques qu'au moins un élément texturant.

### • Décomposition de l'objet de l'image en cellules

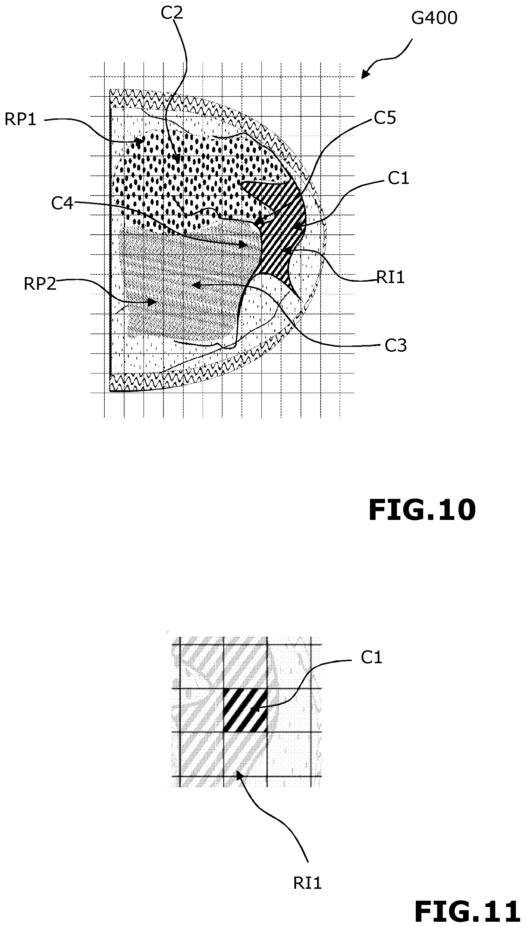

L'image 400 du sein est décomposée en blocs de points adjacents, appelés cellules C1, C2, C3, C4, C5 (Figure 10).

Les cellules issues de la décomposition de l'image résultent de l'application sur l'image d'une grille G400 présentant un maillage de taille prédéfinie.

Initialement, le maillage de la grille, et donc les dimensions des cellules peuvent être choisies de manière à correspondre aux dimensions des plus grands éléments texturants utilisés. Si besoin, une grille présentant un maillage plus fin peut être appliquée par la suite sur l'image ou une partie de l'image, par exemple sur une cellule donnée, pour permettre d'utiliser des éléments texturants de plus petites dimensions.

### • Composition des cellules

On peut prévoir en particulier que lorsqu'une cellule, par exemple la cellule C5 comme illustré à la figure 10, contient des points de différentes catégories de région, ou de différentes sous-catégories, par exemple des points d'une région d'intérêt RI1 et d'une région polluante RP1, RP2, ou encore des points de deux régions polluantes différentes, la cellule correspondante est décomposée en sous-cellules.

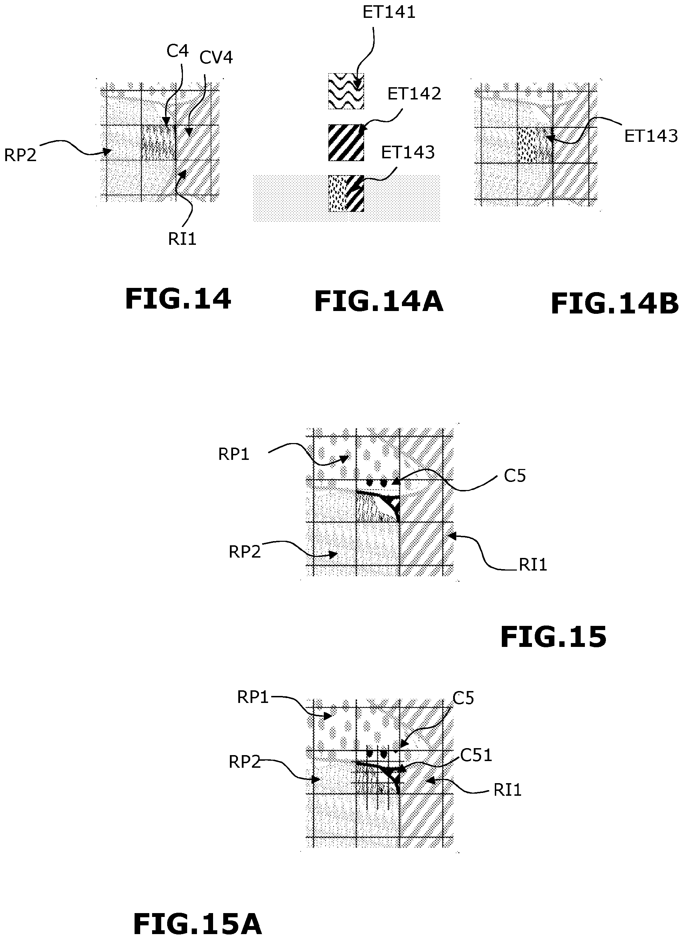

Ainsi, comme illustré à la figure 15A, la cellule C5 est décomposée en sous-cellules C51, qui peuvent être elles-mêmes décomposées en sous-cellules jusqu'à ce chaque sous-cellule contienne uniquement des points de région d'intérêt RI1 ou uniquement des points de région polluante RP1, RP2 (Figures 15 et 15A). Les étapes de traitement présentées dans cette description sont alors appliquées à ces sous-cellules.

La figure 15A illustre ainsi l'application d'un maillage plus fin à la cellule C5 pour que chaque sous-cellule C51 comprenne uniquement des points de région d'intérêt ou uniquement des points de région polluante. On pourra considérer qu'une cellule comprend « uniquement » des points d'une région donnée, lorsque la proportion dans la cellule de ces points appartenant à une région donnée est supérieure à une valeurs seuil donnée, par exemple 95 %.

Autrement dit, la cellule est décomposée suivant une grille avec le maillage inférieur afin d'effectuer une analyse plus fine.

Une telle décomposition peut être graduelle et réalisée plusieurs fois. A chaque nouvelle itération les contenus des cellules sont analysés pour identifier un éventuel besoin de décomposition supplémentaire.

### • Cellule de région d'intérêt

Lorsque chacun des points d'une cellule C1 appartient à une région d'intérêt RI1, lesdits points restent inchangés dans la nouvelle image IMG2 générée (Figure 11). En particulier lesdits points restent inchangés indépendamment du fait que des points des cellules voisines appartiennent à une région polluante ou à une région d'intérêt.

### • Traitement des cellules de région polluante

Lorsque chacun des points d'une cellule appartient à une région polluante et que les points des cellules voisines appartiennent aussi à ladite région polluante, lesdits points de la cellule sont remplacés par les points d'un élément texturant choisi parmi plusieurs éléments texturants prédéfinis.

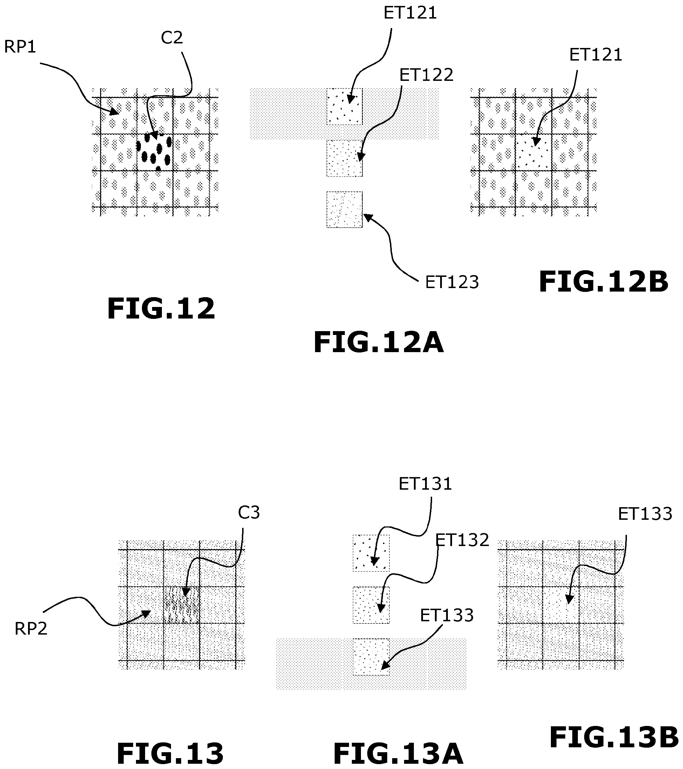

Ainsi comme illustré à la figure 12, la cellule C2 est entourée de cellules voisines qui appartiennent aussi à la région polluante RP1. Le système détermine parmi plusieurs éléments texturants ET121, ET122, ET123, l'élément texturant ET121 qui se rapproche le plus de la cellule C2 sur la base de valeurs d'intensité et des métadonnées associées aux points de la cellule et des éléments texturants. De manière similaire, la cellule C3 illustrée à la figure 13 est entourée de cellules voisines qui appartiennent aussi à la région polluante RP2. Le système détermine parmi plusieurs éléments texturants ET131, ET132, ET133, l'élément texturant ET133 qui se rapproche le plus de la cellule C3 sur la base de valeurs d'intensité et des métadonnées associées aux points de la cellule et des éléments texturants.

Lorsque chacun des points d'une cellule appartient à une région polluante et que des points d'au moins une cellule voisine appartiennent à une catégorie autre que ladite région polluante, par exemple à une région d'intérêt ou à une autre région polluante, un nouvel élément texturant est généré à partir d'au moins un, de préférence de plusieurs, éléments texturants prédéfinis, et en fonction des points de ladite au moins une cellule voisine. On peut prévoir particulier que l'un des éléments texturants servant à générer le nouvel élément texturant soit choisi en fonction desdits points de ladite au moins une cellule voisine. Les points de ladite cellule sont remplacés par les points dudit nouvel élément texturant. Avantageusement, un nouvel élément texturant est généré à partir d'un élément texturant prédéfini choisi en fonction des caractéristiques de ladite cellule à traiter et à partir d'un autre élément texturant choisi en fonction des caractéristiques de ladite au moins une cellule voisine.

Ainsi, comme illustré à la figure 14, chacun des points de la cellule C4 appartient à la région polluante RP1, tandis que la cellule voisine CV4 comprend des points qui appartiennent à la région d'intérêt RI1.

Un nouvel élément texturant ET143 est alors généré à partir de l'élément texturant ET141 prédéfini qui se rapproche de la cellule C4, et de l'élément texturant ET142 qui se rapproche de ou des points de la cellule voisine CV4. Les points de ladite cellule C4 sont alors remplacés par les points dudit nouvel élément texturant ET143 (Figures 14, 14A et 14B). Les points dudit élément texturant ET143 utilisé comme masque pour la cellule C4 peuvent en outre être adaptés en fonction du voisinage de la cellule C4.

### • Image résultante

Comme illustré à la figure 16, la région d'intérêt RI1 de même que la structure géométrique d'intérêt SGI1 qui lui est associée sont conservées, tandis que les régions polluantes RP1, RP2 d'origine ont été modifiées à l'aide des éléments texturants pour fondre ces régions dans leur environnement et ne plus ainsi risquer d'attirer l'attention du radiologue. On peut remarquer dans l'exemple illustré à la figure 16 que la zone de la région référencée RP2 à la figure 5, proche de la région d'intérêt ou de la région polluante référencée RP1 à la figure 5, a été modifiée différemment du reste de la région RP2 afin de conserver la forme et texture générale de l'objet et d'assurer des transitions de régions adaptées de manière à ne pas générer de perturbations visuelles.

Le radiologue peut ainsi concentrer son analyse sur la région d'intérêt RI1 et la structure géométrique d'intérêt SGI1 qui lui est associée.

Les fonctions et étapes décrites peuvent être mise en œuvre sous forme de programme informatique ou via des composants matériels (p. ex. des réseaux de portes programmables). En particulier, les fonctions et étapes opérées par le système 1 informatique peuvent être réalisées par des jeux d'instructions ou modules informatiques implémentés dans un processeur ou contrôleur ou être réalisées par des composants électroniques dédiés ou des composants de type FPGA ou ASIC. Il est aussi possible de combiner des parties informatiques et des parties électroniques.

Lorsqu'il est précisé que le système informatique est configuré pour réaliser une opération donnée, cela signifie que le système comprend des instructions informatiques et les moyens d'exécution correspondants qui permettent de réaliser ladite opération et/ou que le système comprend des composants électroniques correspondant.

L'invention n'est pas limitée aux modes de réalisation illustrés dans les dessins. De plus, le terme « comprenant » n'exclut pas d'autres éléments ou étapes. En outre, des caractéristiques ou étapes qui ont été décrites en référence à l'un des modes de réalisation exposés ci-dessus peuvent également être utilisées en combinaison avec d'autres caractéristiques ou étapes d'autres modes de réalisation exposés ci-dessus, tant que ces combinaisons sont couvertes par les revendications.

## Revendications

1. Procédé mis en œuvre à l'aide d'un système informatique (1) pour le traitement d'au moins une partie, appelée région polluante (RP1, RP2), d'une image (IMG1) numérique d'un objet (3),
l'image étant produite à partir d'un appareil (2) à rayons X par exposition de l'objet (3) à des rayons X,
ladite image étant mémorisée dans une mémoire (103) de données et comprenant :
- des points (P) à chacun desquels est associée une valeur numérique d'intensité, dite intensité initiale I₀, qui correspond à la quantité de photons qui ont frappé un point correspondant de l'objet (3), et
- au moins une métadonnée (M) ladite au moins une métadonnées (M) comprenant au moins l'une des métadonnées suivantes:
- un ou des paramètres physiques de l'objet, tel que l'épaisseur de l'objet,
- un ou des paramètres relatifs à la puissance ou l'énergie des rayons X avec lequel l'image a été acquise, tels que les valeurs de tension et d'intensité électrique utilisées pour produire les rayons X;
ledit procédé comprenant :
- l'identification d'au moins un groupe de points de l'image correspondant à une région polluante (RP1, RP2) en fonction des valeurs d'intensité initiale I₀ des points (P) de l'image et de ladite au moins une métadonnée (M) ;
- la génération d'une nouvelle image (IMG2) comprenant une étape de remplacement des valeurs d'intensité initiale I₀ d'au moins une partie des points de ladite au moins une région polluante (RP1, RP2) par de nouvelles valeurs d'intensité,
**caractérisé**
**en ce que** le procédé comprend la fourniture d'un ensemble de groupes de points, appelés éléments texturants (ET121, ET122, ET123; ET131, ET132, ET133), stockés dans une mémoire de données,
chaque élément texturant comprenant une pluralité de points et des valeurs d'intensité associées aux points,
**en ce que** l'étape de remplacement des valeurs d'intensité initiale I₀ d'au moins une partie des points de ladite au moins une région polluante par de nouvelles valeurs d'intensité comprend :
- la sélection d'un élément texturant parmi plusieurs éléments texturants ou la génération d'un nouvel élément texturant à partir d'un ou de plusieurs éléments texturants sélectionnés,
- le remplacement des valeurs d'intensité initiale I₀ d'au moins une partie des points de ladite au moins une région polluante par les valeurs d'intensité d'au moins une partie des points de l'élément texturant sélectionné ou du nouvel élément texturant généré,
et **en ce que** l'élément texturant sélectionné ou le nouvel élément texturant généré présente une métadonnée correspondant à un type de tissu, par exemple de la graisse, qui est distinct du type de tissu, par exemple un vaisseau sanguin, de la région polluante à remplacer.

2. Procédé selon la revendication 1, **caractérisé en ce que** le procédé comprend aussi l'identification d'au moins un groupe de points de l'image correspondant à une région d'intérêt (RI1) en fonction des valeurs d'intensité initiale I₀ des points (P) de l'image et de ladite au moins une métadonnée (M).

3. Procédé selon l'une des revendications précédentes, **caractérisé en ce que** l'étape d'identification d'au moins un groupe de points de l'image (IMG1) en tant que région polluante (RP1) comprend la comparaison de ces points de l'image avec d'autres groupes de points de référence (PR1, PR2, PR3) mémorisés dans une base de données (BDD), chaque groupe de points de référence étant associé à au moins une métadonnée (MrefPR), dite métadonnée de référence.

4. Procédé selon l'une des revendications précédentes, **caractérisé en ce que** chaque élément texturant comprend au moins une métadonnée et la sélection de l'élément texturant ou la génération du nouvel élément texturant est réalisée en fonction de ladite au moins une métadonnée.

5. Procédé selon l'une des revendications précédentes, **caractérisé en ce que**, pour une région polluante (RP1, RP2) identifiée, le procédé comprend :
- le calcul d'au moins un moment mathématique à partir des valeurs d'intensité, tel que la valeur moyenne d'intensité I_{moy} ou la variance d'intensité, des points d'au moins une partie de ladite région polluante (RP1, RP2) identifiée,
- la sélection d'un groupe de points, appelé élément texturant (ET121 ; ET133), parmi plusieurs éléments texturants (ET121, ET122, ET123; ET131, ET132, ET133) prédéfinis, ou la génération d'un nouvel élément texturant (ET143) à partir d'un ou de plusieurs éléments texturants sélectionnés,
la sélection ou génération d'élément texturant étant réalisée en fonction dudit au moins un moment mathématique calculé, tel que ladite valeur moyenne d'intensité I_{moy} ou ladite variance d'intensité, des points de ladite au moins une partie de région polluante (RP1, RP2), les points dudit élément texturant sélectionné (ET121 ; ET133) ou dudit nouvel élément texturant généré (ET143) présentant un ensemble de valeurs d'intensité données différent de celui des points de ladite au moins une partie de ladite région polluante identifiée ;
et **en ce que** au moins une partie des points dudit élément texturant sélectionné ou dudit nouvel élément texturant généré remplacent les points de ladite au moins une partie de la région polluante identifiée.

6. Procédé selon l'une des revendications précédentes, **caractérisé en ce que** ladite au moins une métadonnée associée à l'image comprend une métadonnée définie par corrélation avec au moins une partie d'une autre image de l'objet, par exemple acquise sous une incidence différente ou à un instant différent.

7. Procédé selon l'une des revendications précédentes, **caractérisé en ce que** ladite étape de remplacement, encore appelée opération de masquage, comprend la décomposition de l'image (400) de l'objet en blocs de points adjacents, appelés cellules (C1, C2, C3, C4, C5).

8. Procédé selon la revendication 7, **caractérisé en ce que**, lorsque chacun des points d'une cellule (C1) appartient à une région d'intérêt (RI1), lesdits points restent inchangés dans la nouvelle image (IMG2) générée.

9. Procédé selon l'une des revendications 7 ou 8, **caractérisé en ce que**, lorsque les points d'une cellule (C2, C3) appartiennent à une région polluante (RP1, RP2) et que les points des cellules voisines appartiennent aussi à ladite région polluante, lesdits points de la cellule (C2) sont remplacés par les points d'un élément texturant (ET121 ; ET133) sélectionné parmi plusieurs éléments texturants (ET121, ET122, ET123 ; ET131, ET132, ET133) prédéfinis.

10. Procédé selon la revendication 9, **caractérisé en ce que** la sélection dudit élément texturant (ET121 ; ET133) est fonction des valeurs d'intensité desdits points de la cellule.

11. Procédé selon l'une des revendications 7 à 10, **caractérisé en ce que**, lorsque les points d'une cellule (C4) appartiennent à une région polluante (RP1) et que des points d'au moins une cellule voisine (CV4) appartiennent à une catégorie autre que ladite région polluante, par exemple à une région d'intérêt ou à une autre région polluante, un nouvel élément texturant (ET143) est généré à partir d'un ou de plusieurs éléments texturants (ET141, ET142) prédéfinis, et en fonction des points de ladite au moins une cellule voisine (CV4), les points de ladite cellule (C4) étant remplacés par les points dudit nouvel élément texturant (ET143).

12. Procédé selon l'une des revendications 7 à 11, **caractérisé en ce que**, lorsqu'une cellule (C5) contient à la fois des points d'une région d'intérêt (RI1) et d'une région polluante (RP1, RP2), la cellule (C5) est décomposée en sous-cellules (C51), qui peuvent être elles-mêmes décomposées en sous-cellules jusqu'à ce chaque sous-cellule contienne uniquement des points de région d'intérêt (RI1) ou uniquement des points de région polluante (RP1, RP2).

13. Procédé selon l'une des revendications précédentes, **caractérisé en ce que** le procédé comprend une étape de traitement de l'image (IMG1) acquise dans laquelle la partie (400) de l'image correspondant à l'objet (3), appelée image (400) de l'objet, est isolée par rapport au fond (500) de l'image acquise.

14. Procédé selon l'une des revendications précédentes, **caractérisé en ce que** le procédé comprend une étape de détermination d'une région, dite région de contour (RC), correspondant à une zone de contour de l'objet (3).

15. Procédé selon l'une des revendications précédentes, **caractérisé en ce que** le procédé comprend l'identification, dans la partie (400) d'image correspondant à l'objet, d'au moins une structure géométrique (SGI1, SGP1), par exemple une structure linéaire,
et **en ce que** le procédé comprend l'attribution à ladite au moins une structure géométrique identifiée (SGI1, SGP1) d'un paramètre de classification présentant une première valeur, correspondant à une catégorie d'intérêt, lorsqu'une corrélation est établie entre ladite structure géométrique et au moins une région d'intérêt; et une deuxième valeur, correspondant à une catégorie de non-intérêt, lorsqu'il n'existe pas de corrélation entre ladite structure géométrique et la ou les régions d'intérêt.

16. Procédé selon l'une des revendications précédentes, **caractérisé en ce que**, l'objet (3) étant comprimable sous l'effet d'une force de compression donnée, ladite au moins une métadonnée (M) comprend l'épaisseur de l'objet à l'état comprimé et la force de compression appliquée à l'objet.

17. Produit de programme informatique non transitoire comprenant des instructions de code de programme pour l'exécution des étapes d'un procédé conforme à l'une des revendications précédentes, lorsque ledit programme est exécuté par un processeur (102) d'un système informatique (1).

18. Système informatique (1) comprenant une mémoire (101) contenant des instructions de code de programme pour l'exécution des étapes d'un procédé conforme à l'une des revendications 1 à 16, un processeur (102) permettant d'exécuter lesdites instructions de code de programme, une mémoire (103) permettant de mémoriser des données d'image (D) et des métadonnées (M), et de préférence un écran d'affichage (104).

19. Installation comprenant un système informatique (1) selon la revendication 18, et un appareil (2) à rayons X, ledit appareil (2) comprenant un dispositif émetteur (210) apte à émettre un faisceau de rayons X et un dispositif récepteur (220) apte à recevoir les rayons X émis par le dispositif émetteur (210) après leur passage à travers l'objet (3),
et l'appareil (2) à rayons X étant configuré pour transmettre au système informatique (1) des données d'image (D), correspondant aux rayons X reçus par le dispositif récepteur (202) après leur passage à travers l'objet (3), et, éventuellement, des métadonnées (M) comprenant par exemple un ou des paramètres relatifs à la puissance ou à l'énergie des rayons X produits.

## Patentansprüche

1. Unter Zuhilfenahme eines Rechensystems (1) auszuführendes Verfahren zur Verarbeitung mindestens eines Teils ("Störregion") (RP1, RP2) eines digitalen Bildes (IMG1) eines Gegenstandes (3),
wobei das Bild durch eine Röntgeneinrichtung (2) durch Belichtung des Gegenstandes (3) mit Röntgenstrahlen erzeugt wird,
wobei das Bild in einem Datenspeicher (103) gespeichert wird und umfasst:
- Punkte (P), jedem von denen ein Intensitätszahlenwert ("Ausgangsintensität" I0) zuegordnet ist, der der Photonenmenge, die auf einen entsprechenden Punkt am Gegenstand (3) getroffen hat, entspricht,
- mindestens ein Metadatum (M),
wobei das mindestens eine Metadatum (M) mindestens eines folgender Metadaten umfasst:
- mindestens einen physikalischen Parameter des Gegenstandes, wie z.B. die Dicke des Gegenstandes,
- mindestens einen die Leistung oder Energie der Röntgenstrahlen, mit denen das Bild aufgenommen wurde, betreffenden Parameter, wie z.B. die zur Erzeugung der Röntgenstrahlen verwendete Spannungs- und Stromstärkewerte;
wobei das Verfahren umfasst:
- Erkennen mindestens eines Punktsatzes des Bildes, die einer Störregion (RP1, RP2) entsprechen, als Funktion der Ausgangsidentitätswerte I₀ der Punkte (P) des Bildes und des mindestens einen Metadatums (M);
- Erzeugen eines neuen Bildes (IMG2), umfassend Ersetzen der Ausgangsintensitätswerte I₀ mindestens eines Teils der Punkte der mindestens einen Störregion (RP1, RP2) durch neue Intensitätswerte,
**dadurch gekennzeichnet, dass** das Verfahren umfasst: Vorsehen einer Gruppe von Punktsätzen ("strukturgebende Elemente") (ET121, ET122, ET123; ET131, ET132, ET133), die in einem Datenspeicher gespeichert sind,
wobei jedes strukturgebende Element eine Vielzahl Punkte und die diesen zugeordneten Intensitätswerte umfasst,
dass das Ersetzen der Ausgangsintensitätswerte I₀ mindestens eines Teils der Punkte der mindestens einen Störregion durch neue Intensitätswerte umfasst:
- Auswählen eines strukturgebenden Elementes aus mehreren strukturgebenden Elementen oder Erzeugen eines neuen strukturgebenden Elementes aus mindestens einem ausgewählten strukturgebenden Element,
- Ersetzen der Ausgangsintensitätswerte I₀ mindestens eines Teils der Punkte der mindestens einen Störregion durch die Intensitätswerte mindestens eines Teils der Punkte des ausgewählten bzw. neu erzeugten strukturgebenden Elementes,
und dass das ausgewählte bzw. neu erzeugte strukturgebende Element ein Metadatum aufweist, das einer Gewebeart, z.B. Fett, entspricht, die sich von der Gewebeart, z.B. Blutgefäß, der zu ersetzenden Störregion unterscheidet.

2. Verfahren nach Anspruch 1, **dadurch gekennzeichnet, dass** das Verfahren ferner umfasst: Erkennen mindestens eines Punktsatzes des Bildes, die einer interessierenden Region (RI1) entsprechen, als Funktion der Ausgangsidentitätswerte I₀ der Punkte (P) des Bildes und des mindestens einen Metadatums (M).

3. Verfahren nach einem der vorstehenden Ansprüche, **dadurch gekennzeichnet, dass** das Erkennen mindestens eines Punktsatzes des Bildes (IMG1) als Störregion (RP1) umfasst: Vergleichen dieser Punkte des Bildes mit anderen in einer Datenbank (BDD) gespeicherten Referenzpunktsätzen (PR1, PR2, PR3), wobei jeder Referenzpunktsatz mindestens einem Metadatum (MrefPR) ("Referenzmetadatum") zugeordnet ist.

4. Verfahren nach einem der vorstehenden Ansprüche, **dadurch gekennzeichnet, dass** jedes strukturgebende Element mindestens ein Datum umfasst und das Auswählen des strukturgebenden Elementes bzw. das Erzeugen des neuen strukturgebenden Elementes als Funktion des mindestens einen Metadatums erfolgt.

5. Verfahren nach einem der vorstehenden Ansprüche, **dadurch gekennzeichnet, dass** das Verfahren für eine erkannte Störregion (RP1, RP2) umfasst:
- Berechnen mindestens eines mathematischen Momentes anhand der Intensitätswerte, z.B. des Intensitätsmittelwerts I_{moy} oder der Intensitätsvarianz der Punkte mindestens eines Teils der erkannten Störregion (RP1, RP2),
- Auswählen eines Punktsatzes ("strukturgebendes Element") (ET121; ET133) aus mehreren vorgegebenen strukturgebenden Elementen (ET121, ET122, ET123; ET131, ET132, ET133) oder Erzeugen eines neuen strukturgebenden Elementes (ET143) aus mindestens einem ausgewählten strukturgebenden Element,
wobei das Auswählen bzw. das Erzeugen eines strukturgebenden Elementes als Funktion mindestens eines berechneten mathematischen Momentes, z.B. des Intensitätsmittelwerts I_{moy} oder der Intensitätsvarianz, der Punkte des mindestens einen Teils einer Störregion (RP1, RP2) erfolgt, wobei die Punkte des ausgewählten (ET121; ET133) bzw. neu erzeugten strukturgebenden Elementes (ET143) einen Satz gegebener Intensitätswerte aufweisen, die sich von denen der Punkte des mindestens einen Teils der erkannten Störregion unterscheiden; und
dass mindestens ein Teil der Punkte des ausgewählten bzw. neu erzeugten strukturgebenden Elementes an die Stelle der Punkte des mindestens einen Teils der erkannten Störregion treten.

6. Verfahren nach einem der vorstehenden Ansprüche, **dadurch gekennzeichnet, dass** das dem Bild zugeordnete mindestens eine Metadatum ein durch Korrelation mit mindestens einem Teil eines anderen, z.B. in einem anderen Einfallswinkel oder zu einem anderen Zeitpunkt aufgenommenen, Bildes des Gegenstandes definiertes Metadatum umfasst.

7. Verfahren nach einem der vorstehenden Ansprüche, **dadurch gekennzeichnet, dass** das Ersetzen ("Maskieren") umfasst: Zerlegen des Bildes (400) des Gegenstandes in Blöcke aus aneinander angrenzenden Punkten ("Zellen") (C1, C2, C3, C4, C5).

8. Verfahren nach Anspruch 7, **dadurch gekennzeichnet, dass**, wenn jeder der Punkte einer Zelle (C1) zu einer interessierenden Region (RI1) gehört, die Punkte im neu erzeugten Bild (IMG2) unverändert bleiben.

9. Verfahren nach einem der Ansprüche 7 oder 8, **dadurch gekennzeichnet, dass**, wenn die Punkte einer Zelle (C2, C3) zu einer Störregion (RP1, RP2) gehören und die Punkte der benachbarten Zellen ebenfalls zur Störregion gehören, die Punkte der Zelle (C2) durch die Punkte eines aus mehreren vorgegebenen strukturgebenden Elementen (ET121, ET122, ET123; ET131, ET132, ET133) ausgewählten strukturgebenden Elementes (ET121, ET123) ersetzt werden.

10. Verfahren nach Anspruch 9, **dadurch gekennzeichnet, dass** das strukturgebende Element (ET121; ET133) als Funkction der Intensitätswerte der Punkte der Zelle ausgewählt wird.

11. Verfahren nach einem der Ansprüche 7 -10, **dadurch gekennzeichnet, dass**, wenn die Punkte einer Zelle (C4) zu einer Störregion (RP1) gehören und die Punkte mindestens einer benachbarten Zelle (CV4) zu einer anderen Kategorie gehören als Störregion, z.B. zu einer interessierenden Region oder einer anderen Störregion, ein neues strukturgebendes Element (ET143) anhand mindestens eines vorgegebgenen strukturgebenden Elementes (ET141, ET142) und als Funktion der Punkte der mindestens einen benachbarten Zelle (CV4) erzeugt wird, wobei die Punkte der Zelle (C4) durch die Punkte des neuen strukturgebenden Elementes (ET143) ersetzt werden.

12. Verfahren nach einem der Ansprüche 7 - 11, **dadurch gekennzeichnet, dass**, wenn eine Zelle (C5) Punkte einer interessierenden Region (RI1) und einer Störregion (RP1, RP2) zugleich enthält, die Zelle (C5) in Unterzellen (C51) zerlegt wird, die sich wiederum solange in Unterzellen zerlegen lassen, bis jede Unterzelle ausschließlich Punkte einer interessierenden Region (RI1) oder ausschließlich Punkte einer Störregion (RP1, RP2) enthält.

13. Verfahre nach einem der vorstehenden Ansprüche, **dadurch gekennzeichnet, dass** das Verfahren umfasst: Verarbeiten des aufgenommenen Bildes (IMG1), wobei der dem Gegenstand (3) entsprechende Teil des Bildes ("Bild (400) des Gegenstandes") vom Hintergrund (500) des aufgenommenen Bildes isoliert wird.

14. Verfahren nach einem der vorstehenden Ansprüche, **dadurch gekennzeichnet, dass** das Verfahren umfasst: Bestimmen einer Region ("Konturregion") (RC), die einem Konturbereich des Gegenstandes (3) entspricht.

15. Verfahren nach einem der vorstehenden Ansprüche, **dadurch gekennzeichnet, dass** das Verfahren umfasst: Erkennen mindestens einer geometrischen Struktur (SGI1, SGP1), z.B. einer linearen Struktur, in einem dem Gegenstand entsprechenden Teil (400) des Bildes, und dass das Verfahren umfasst: Zuweisen eines Einstufungsparameters mit einem einer interessierenden Kategorie entsprechenden ersten Wert zu einer erkannten geometrischen Struktur (SGI1, SGP1), wenn eine Korrelation zwischen der geometrischen Struktur und mindestens einer interessierenden Region hergestellt wird, und mit einem einer nicht interessierenden Kategorie entsprechenden zweiten Wert, wenn keine Korrelation zwischen der geometrischen Struktur und der mindestens einen interessierenden Region vorliegt.

16. Verfahren nach einem der vorstehenden Ansprüche, **dadurch gekennzeichnet, dass**, da der Gegenstand (3) unter der Einwirkung einer bestimmten Druckkraft komprimierbar ist, das mindestens eine Metadatum (M) die Dicke des komprimierten Gegenstandes und die auf den Gegenstand angewandte Druckkraft umfasst.

17. Nicht flüchtiges Computerprogrammprodukt, umfassend Programmcodebefehle zur Ausführung der Schritte eines Verfahrens nach einem der vorstehenden Ansprüche bei Ausführung des Programms durch einen Prozessor (102) eines Rechensystems (1).

18. Rechensystem (1), umfassend einen Speicher (101), der Programmcodebefehle zur Ausführung der Schritte eines Verfahrens nach einem der Ansprüche 1 - 16 enthält, einen Prozessor (102), der die Ausführung der Programmcodebefehle ermöglicht, einen Speicher (103), der die Speicherung von Bilddaten (D) und Metadaten (M) ermöglicht, und vorzugsweise einen Bildschirm (104).

19. Einrichtung, umfassend ein Rechensystem (1) nach Anspruch 18 und eine Röntgeneinrichtung (2), wobei die Einrichtung (2) eine zur Ausstrahlung eines Röntgenstrahls geeigneten Emissionsvorrichtung (210) und eine Empfangsvorrichtung (210), die dazu geeignet ist, die von der Emissionsvorrichtung (210) ausgestrahlten Röntgenstrahlen zu empfangen, nachdem sie den Gegenstand (3) durchlaufen haben,
und wobei die Röntgeneinrichtung (2) derart konfiguriert ist, dass sie dem Rechensystem (1) Bilddaten (D), die den Röntgenstrahlen entsprechen, die von der Empfangsvorrichtung (202) empfangen wurden, nachdem diese den Gegenstand (3) durchlaufen haben, und ggf. Metadaten (M), die z.B. mindestens einen die Leistung oder der Energie der erzeugten Röntgenstrahlen betreffenden Parameter umfassen, übersendet.

## Claims

1. Method, using a computer system (1), for processing at least one part ('polluting region') (RP1, RP2) of a digital image (IMG1) of an object (3),
wherein the image is produced by an X-ray machine (2) by exposing the object (3) to X-rays,
wherein the image is stored in a data memory (103) and comprises:
- points (P), each of which is associated with a numerical intensity value ('initial intensity') I₀, which corresponds to the quantity of photons that have hit a corresponding point of the object (3), and
- at least one metadatum (M)
wherein the at least one metadatum (M) comprises at least one of the following metadata:
- one or more physical parameters of the object, such as the thickness of the object,
- one or more parameters related to the power or energy of the X-rays with which the image was obtained, such as the voltage and current density used to produce the X-rays;
wherein the method comprises:
- identifying at least one group of points of the image corresponding to a polluting region (RP1, RP2) as a function of the initial intensity I₀ values of the points (P) of the image and the at least one metadatum (M);
- generating a new image (IMG2) comprising a step of replacing the initial intensity values I₀ of at least some of the points of the at least one polluting region (RP1, RP2) with new intensity values,
**characterised in that** the method comprises providing a set of groups of points ('texturing elements') (ET121, ET122, ET123; ET131, ET132, ET133) stored in a data memory, wherein each texturing element comprises a plurality of points and intensity values associated with the points,
**in that** the step of replacing the initial intensity values I₀ of at least some of the points of the at least one polluting region with new intensity values comprises:
- - selecting a texturing element from several texturing elements or generating a new texturing element from one or more selected texturing elements,
- replacing the initial intensity values I₀ of at least some of the points of the at least one polluting region with the intensity values of at least some of the points of the texturing element selected or the new texturing element generated,
**in that** the texturing element selected or the new texturing element generated has a metadatum corresponding to a type of tissue, e.g. fat, that is distinct from the type of tissue, e.g. a blood vessel, of the polluting region being replaced.

2. Method according to claim 1, **characterised in that** the method also comprises identifying at least one group of points of the image corresponding to a region of interest (RM) as a function of the initial intensity I₀ values of the points (P) of the image and the at least one metadatum (M).

3. Method according to any of the foregoing claims, **characterised in that** the step of identifying at least one group of points of the image (IMG1) as a polluting region (RP1) comprises comparing these points of the image with other groups of reference points (PR1, PR2, PR3) stored in a database (BDD), wherein each group of reference points is associated with at least one metadatum (MrefPR) ('reference metadatum').

4. Method according to any of the foregoing claims, **characterised in that** each texturing element comprises at least one metadatum, and the selection of the texturing element or the generation of the new texturing element is carried out as a function of the at least one metadatum.

5. Method according to any of the foregoing claims, **characterised in that**, for a polluting region (RP1, RP2) identified, the method comprises:
- calculating at least one mathematical moment from the intensity values, such as the mean intensity value I_{moy} or the intensity variance, of the points of at least part of the polluting region (RP1, RP2) identified,
- selecting a group of points ('texturing element') (ET121; ET133) from several predefined texturing elements (ET121, ET122, ET123; ET131, ET132, ET133) or generating a new texturing element (ET143) from one or more selected texturing elements,
wherein the selection or generation of the texturing element is carried out as a function of the at least one calculated mathematical moment, such as the mean intensity value Imoy or the intensity variance, of the points of the at least one polluting region (RP1, RP2), wherein the points of the selected texturing element (ET121; ET133) or the new texturing element generated (ET143) have a set of given intensity values different to that of the points of the at least one part of the identified polluting region; and
**in that** at least some of the points of the texturing element selected or the new texturing element generated replace the points of the at least one part of the identified polluting region.

6. Method according to any of the foregoing claims, **characterised in that** the at least one metadatum associated with the image comprises a metadatum defined by correlation with at least one part of another image of the object, e.g. obtained at a different incidence or at a different moment.

7. Method according to any of the foregoing claims, **characterised in that** the step of replacing (also 'masking operation') comprises breaking down the image (400) of the object into blocks of adjacent points ('cells)' (C1, C2, C3, C4, C5).

8. Method according to claim 7, **characterised in that**, when each of the points of a cell (C1) belongs to a region of interest (RM), the points remain unchanged in the new image (IMG2) generated.

9. Method according to either of claims 7 or 8, **characterised in that**, when the points of a cell (C2, C3) belong to a polluting region (RP1, RP2) and the points of the adjacent cells also belong to the polluting region, the points of the cell (C2) are replaced with the points of a texturing element (ET121; ET133) selected from several predefined texturing elements (ET121, ET122, ET123; ET131, ET132, ET133).

10. Method according to claim 9, **characterised in that** the selection of the texturing element (ET121; ET133) is a function of the intensity values of the points of the cell.

11. Method according to any of claims 7 - 10, **characterised in that**, when the points of one cell (C4) belong to a polluting region (RP1) and the points of at least one adjacent cell (CV4) belong to a category other than that of the polluting region, e.g. a region of interest or another polluting region, a new texturing element (ET143) is generated from one or more predefined texturing elements (ET141, ET142), and as a function of the points of the at least one adjacent cell (CV4), wherein the points of the cell (C4) are replaced by the points of the new element (ET143).

12. Method according to any of claims 7 - 11, **characterised in that**, when a cell (C5) simultaneously contains points of a region of interest (RI1) and of a polluting region (RP1, RP2), the cell (C5) is broken down into subcells (C51), which may themselves be broken down into subcells until each subcell solely contains points of the region of interest (RI1) or only contains points of the polluting region (RP1, RP2).

13. Method according to any of the foregoing claims, **characterised in that** the method comprises a step of processing the image (IMG1) obtained, wherein the part (400) of the image corresponding to the object (3) (image (400) of the object') is isolated from the background (500) of the image obtained.

14. Method according to any of the foregoing claims, **characterised in that** the method comprises a step of determining a region ('contour region') (RC) corresponding to a contour area of the object (3).

15. Method according to any of the foregoing claims, **characterised in that** the method comprises identifying at least one geometrical structure (SGI1, SGP1), e.g. a linear structure, within the part of the image (400) corresponding to the object,
and **in that** the method comprises attributing to the at least one geometrical structure identified (SG11, SGP1) a classification parameter having a first value, corresponding to a category of interest, when a correlation is established between the geometrical structure and at least one region of interest; and a second value, corresponding to an area that is not of interest, when there is no correlation between the geometrical structure and the region(s) of interest.

16. Method according to any of the foregoing claims, **characterised in that**, given that the object (3) can be compressed under a given compression force, the at least one metadatum (M) comprises the thickness of the object when compressed and the compression force applied to the object.

17. Non-transitory computer program product comprising program code instructions for carrying out the steps of a method according to any of the foregoing claims when run by a processor (102) of a computer system (1).

18. Computer system (1) comprising a memory (101) containing program code instructions for carrying out the steps of the method according to any of claims 1 - 16, a processor (102) allowing for the running of the program code instructions, a memory (103) allowing for the storage of image data (D) and metadata (M), and, preferably, a display screen (104).

19. Installation comprising a computer system (1) according to claim 18 and an X-ray machine (2), wherein the machine (2) comprises an emitter device (210) suited to emit an X-ray and a receiver device (220) suited to receive the X-rays emitted by the emitter device (210) after they have passed through the object (3),
and wherein the X-ray machine (2) is configured to transmit to the computer system (1) image data (D) corresponding to the X-rays received by the receiver device (202) after they have passed through the object (3) and, optionally, metadata (M), e.g. comprising one or more parameters related to the power or energy of the X-rays produced.
